# FASCICULE DE BREVET EUROPEEN

(11) **EP 0 966 947 B1**
(45) Date de publication et mention de la délivrance du brevet: **14.11.2001**
(21) Numéro de dépôt: 99401431.4
(22) Date de dépôt: 11.06.1999
(51) Int. Cl.: A61K 7/06

(54) **Composition cosmétique contenant un polymère cationique et un terpolymère acrylique et utilisation de cette composition pour le traitement des matières kératiniques**
Kosmetische Zusammensetzung enthaltend ein kationisches Polymer und ein acrylisches Terpolymer und Verwendung dieser Zusammensetzung zur Behandlung von Keratinfasern
Cosmetic composition containing a cationic polymer and an acrylic terpolymer and use of said composition for treating keratinic materials

(30) Priorité: 15.06.1998 FR 9807513
(43) Date de publication de la demande: 29.12.1999
(73) Titulaire: L'OREAL, 75008 Paris (FR)
(72) Inventeur: DUPUIS, Christine, 75018 Paris (FR)
(74) Mandataire: Casalonga, Axel

(56) Documents cités:
- EP-A- 0 824 914

## Description

La présente invention concerne des compositions cosmétiques contenant en association, au moins un polymère cationique et un terpolymère acrylique, ainsi que l'utilisation de ces compositions pour le traitement des matières kératiniques, en particulier les cheveux.

Les polymères cationiques sont utilisés pour donner aux cheveux de bonnes propriétés cosmétiques telles que la douceur, le. toucher et l'aptitude au démêlage.

Il est intéressant de formuler des compositions capillaires contenant des polymères cationiques, qui aient une viscosité élevée et se présentent sous une forme liquide épaissie s'étalant bien telle qu'une crème ou un gel de soin ou de coiffage, très appréciée du consommateur car elle ne coule pas sur le front, la nuque, le visage ou dans les yeux.

Pour cela, on fait en général appel à des polymères épaississants et/ou gélifiants. Cependant, l'introduction des polymères cationiques dans les épaississants conduit souvent à des problèmes de fluidification et de perte de limpidité et les performances cosmétiques obtenues sont parfois insuffisantes pour des produits de soin.

On connaît des polymères épaississants et/ou gélifiants comportant dans leur chaîne une partie hydrophile et une partie hydrophobe constituée d'une chaîne grasse comme le produit "PEMULEN TR1" commercialisé par la société GOODRICH ou les polymères "ACRYSOL" commercialisés par la société ROHM & HAAS. Le polymère "PEMULEN TR1", utilisé en association avec des polymères cationiques, ne conduit pas à un gel de texture satisfaisante et ne donne pas sur le plan cosmétique des résultats satisfaisants, notamment en ce qui concerne le pouvoir fixant, la douceur et le toucher. Le polymère "ACRYSOL 44", utilisé en association avec un polymère cationique, conduit à un produit liguide et trouble.

On connaît aussi par la demande EP-A-0 824 914 des polymères épaississants ou modificateurs de rhéologie comprenant
a) un monomère acrylate ou méthacrylate,
b) un monomère hétérocyclique vinyl-substitué, méthacrylamide, alkylaminoalkyl(méth)acrylate ou alkylaminoalkyl(méth)acrylamide et éventuellement
c) un monomère associatif pouvant être, entre autres, un uréthane non-ionique provenant de la réaction d'un tensio-actif non-ionique monohydrique avec un monoisocyanate insaturé monoéthylénique.

La demanderesse a decouvert de manière surprenante qu'en utilisant une nouvelle famille de polymères épaississants et/ou gélifiants et en les associant à des polymères cationiques, on pouvait obtenir des formulations cosmétiques présentant une viscosité satisfaisante à un pH relativement peu élevé, non pâteuses, non grasses, qui s'étalent bien et qui confèrent aux cheveux de bonnes propriétés de douceur, toucher et démêlage tout en ayant de bonnes propriétés fixantes.

La présente invention a donc pour objet des compositions cosmétiques contenant, dans un support aqueux cosmétiquement acceptable, au moins un polymère cationique et un terpolymère acrylique que l'on définira plus en détail dans la suite de la description.

Ce polymère permet notamment de préparer des compositions aqueuses ou hydro-organiques contenant des solvants cosmétiquement acceptables, rincées ou non rincées, épaissies ou gélifiées.

Les avantages de ce terpolymère sont d'être stable en milieu électrolyte et d'avoir un très bon pouvoir épaississant à pH égal ou supérieur à 5,5 permettant d'atteindre un bon niveau de viscosité et de pouvoir utiliser des concentrations élevées en alcool.

Le terpolymère acrylique utilisé conformément à l'invention est soluble ou gonflable dans les alcalis. Il est caractérisé par le fait qu'il comprend :
a) 20 à 70% en poids, de préférence 25 à 55% en poids, d'un acide carboxylique à insaturation α, β-monoéthylénique ;
b) 20 à 80% en poids, de préférence 30 à 65% en poids, d'un monomère à insaturation monoéthylénique non tensio-actif différent de a) et
c) 0,5 à 60% en poids, de préférence 10 à 50% en poids, d'un monomère uréthane non-ionique qui est le produit de réaction d'un tensio-actif non-ionique monohydrique avec un monoisocyanate à insaturation monoéthylénique.

L'acide carboxylique à insaturation α, β-monoéthylénique a) peut être choisi parmi de nombreux acides et en particulier l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'amino méthyl propanol ou l'amino méthyl propanediol.

Le terpolymère doit aussi contenir une proportion importante indiquée ci-dessus d'un monomère b) à insaturation monoéthylénique qui n'a pas de propriété tensio-active. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensio-actifs non-ioniques monohydriques utilisés pour obtenir le monomère uréthane non-ionique c) sont bien connus et sont généralement des composés hydrophobes alcoxylés contenant un oxyde d'alkylène formant la partie hydrophile de la molécule. Les hydrophobes sont généralement constitués par un alcool aliphatique ou un alkylphénol dans lesquels une chaîne carbonée contenant au moins six atomes de carbone constitue la partie hydrophobe du tensio-actif.

Les tensio-actifs non ioniques monohydriques préférés ont pour formule : 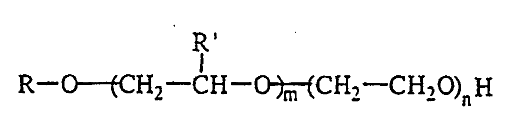 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R' préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Le monoisocyanate monoéthylénique préféré est l'α,α-diméthyl-m-isopropényl-benzylisocyanate.

Le terpolymère acrylique défini ci-dessus est obtenu par copolymérisation en émulsion aqueuse des composants a), b) et c) qui est tout à fait usuelle et décrite dans la demande de brevet EP-A-0 173 109.

A titre de terpolymères pouvant être utilisés selon l'invention, on peut citer les produits de réaction de l'acide méthacrylique comme composant a), de l'acrylate d'éthyle comme composant b) et d'un macromonomère uréthane non-ionique comme composant c), ayant la structure suivante : 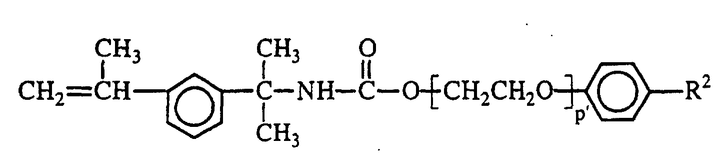 dans laquelle p' va de 6 à 150 et est égal de préférence à 30 et R² est un radical alkyle en C₈-C₁₃, tels que celui décrit dans l'exemple 3 de la demande de brvet EP-A-0 173 109.

Le terpolymère acrylique préféré utilisé selon l'invention est obtenu à partir d'acide méthacrylique comme composant a), d'acrylate de méthyle comme composant b) et d'un macromonomère uréthane non-ionique comme composant (c), ayant la structure suivante : 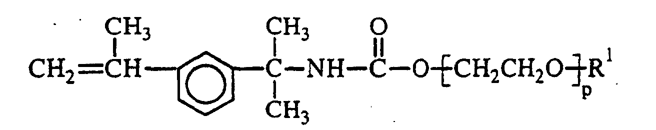 dans laquelle p va de 6 à 150 et R¹ est un radical alkyle en C₁₈-C₂₆, de préférence en C₂₀-C₂₄ linéaire, d'origine végétale tel que le radical docosyle.

Le terpolymère acrylique est présent dans les compositions cosmétiques de l'invention dans des concentrations allant de 0,01 à 20% en poids par rapport au poids total de la composition et préférentiellement de 0,1 à 10% en poids.

Les polymères cationiques utilisables conformément à la présente invention peuvent être choisis parmi tous ceux déjà connus en soi notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire comprise entre 500 et 5.10⁶ environ, et de préférence comprise entre 10³ et 3.10⁶ environ .

Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "QUAT-PRO E" par la Société MAYBROOK et dénommés dans le dictionnaire CTFA "TRIETHONIUM HYDROLYZED COLLAGEN ETHOSULFATE";
- les hydrolysats de collagène portant des groupements chlorure de triméthylammonium et de triméthylstéarylammonium, vendus sous la dénomination de "QUAT-PRO S" par la Société MAYBROOK et dénommés dans le dictionnaire CTFA "STEARTRIMONIUMHYDROLYZEDCOLLAGEN";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres:
- le "CROQUAT L" dont les groupements ammonium quaternaires comportent un groupement alkyle en C₁₂;
- le "CROQUAT M" dont les groupements ammonium quaternaires comportent des groupements alkyle en C₁₀-C₁₈;
- le "CROQUAT S" dont les groupements ammonium quaternaires comportent un groupement alkyle en C₁₈;
- le "CROTEIN Q" dont les groupements ammonium quaternaires comportent au moins un groupe alkyle ayant de 1 à 18 atomes de carbone.

Ces différents produits sont vendus par la Société CRODA.

D'autre protéines ou hydrolysats quaternisés sont par exemple ceux répondant à la formule: 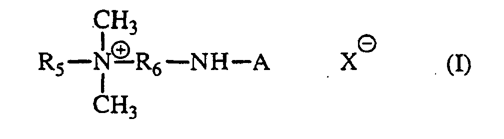 dans laquelle X^{⊖}est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société INOLEX, sous la dénomination "LEXEIN QX 3000", appelé dans le dictionnaire CTFA "COCOTRIMONIUM COLLAGEN HYDROLYSATE".

On peut encore citer les protéines végétales quaternisées telles que les protéines de blé, de maïs ou de soja: comme protéines de blé quaternisées, on peut citer celles commercialisées par la Société CRODA sous les dénominations "HYDROTRITICUM WQ ou QM", appelées dans le dictionnaire CTFA "COCODIMONIUM HYDROLYSED WHEAT PROTEIN", "HYDROTRITICUM QL" appelée dans le dictionnaire CTFA "LAURIDIMONIUM HYDROLYSED WHEAT PROTEIN", ou encore "HYDROTRITICUM QS", appelée dans le dictionnaire CTFA "STEARDIMONIUM HYDROLYSED WHEATPROTEIN".

Une autre famille de polymères cationiques est celle des polymères cationiques siliconés. Parmi ces polymères, on peut citer:
(a) les polymères siliconés répondant à la formule (II) suivante: 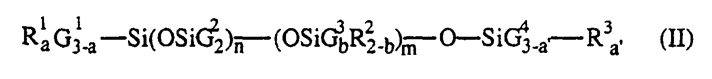 dans laquelle:
G¹, G², G³ et G⁴ identiques ou différents, désignent un atome d'hydrogène, un groupement phényle, OH, alkyle en C₁-C₁₈, par exemple méthyle, alcényle en C₂-C₁₈ ou alcoxy en C₁-C₁₈
a, a', identiques ou différents, désignent le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10,
R₁, R₂, R₃ et R₄, identiques ou différents, désignent un radical monovalent de formule -C_{q}H_{2q}Oₛ R⁵ₜL dans laquelle q est un nombre de 1 à 8, s et t, identiques ou différents, sont égaux à 0 ou à 1, R⁵ désigne un groupement alkylène éventuellement hydroxylé et L est un groupement aminé éventuellement quaternisé choisi parmi les groupements:

   ―NR"―CH₂―CH₂―N'(R")₂

   ―N(R")₂

   ―N^{⊕}(R")₃A^{⊖}

   ―N^{⊕}H(R")₂A^{⊖}

   ―N^{⊕}H₂(R")A^{⊖}

   ―N(R")―CH₂―CH₂―N^{⊕}R"H₂A^{⊖},
dans lesquels R" peut désigner hydrogène, phényle, benzyle, ou un radical hydrocarboné saturé monovalent, par exemple un radical alkyle ayant de 1 à 20 atomes de carbone et A^{⊖} représente un ion halogénure tel que par exemple fluorure, chlorure, bromure ou iodure.

Des produits correspondant à cette définition sont par exemple les polysiloxanes dénommés dans le dictionnaire CTFA "AMODIMETHICONE" et répondant à la formule (II) suivante: 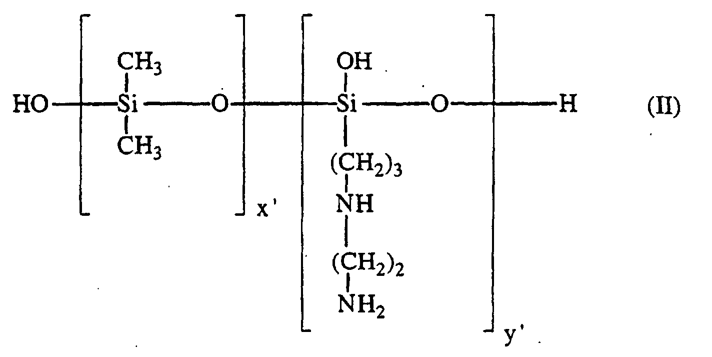 dans laquelle x' et y' sont des nombres entiers dépendant du poids moléculaire, généralement tels que ledit poids moléculaire est compris entre 5 000 et 20 000 environ.

Un produit correspondant à la formule (II) est le polymère dénommé dans le dictionnaire CTFA "TRIMETHYLSILYLAMODIMETHICONE", répondant à la formule: 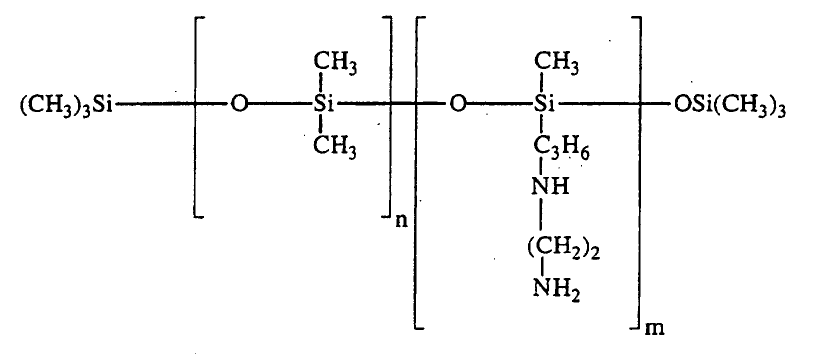 dans laquelle n et m ont les significations données ci-dessus (cf. formule II).

Un produit commercial répondant à cette définition est un mélange (90/10 en poids) d'un polydiméthylsiloxane à groupements aminoéthyl aminoisobutyle et d'un polydiméthylsiloxane vendu sous la dénomination "Q2-8220" par la société DOW CORNING.

De tels polymères sont décrits par exemple dans la demande de brevet EP-A-95238.

D'autres polymères répondant à la formule (II) sont les polymères siliconés répondant à la formule suivante: 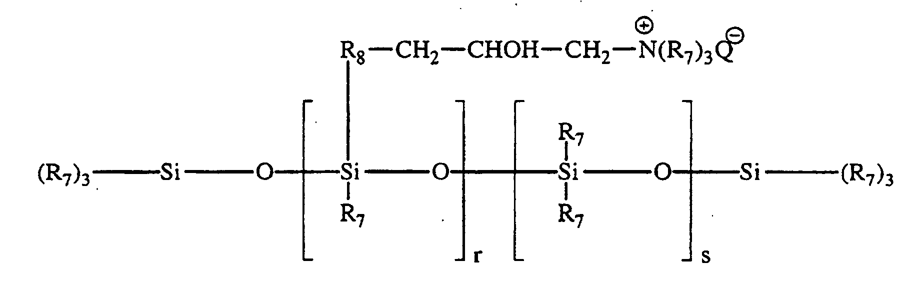 dans laquelle:
R₇ représente un radical hydrocarboné monovalent ayant de 1 à 18 atomes de carbone, et en particulier un radical alkyle en C₁-C₁₈, ou alcényle en C₂-C₁₈, par exemple méthyle; R₈ représente un radical hydrocarboné divalent, notamment un radical alkylène en C₁-C₁₈ ou un radical alkylèneoxy divalent en C₁-C₁₈, par exemple en C₁-C₈;
Q^{⊖} est un ion halogénure, notamment chlorure;
r représente une valeur statistique moyenne de 2 à 20 et en particulier de 2 à 8 ;
s représente une valeur statistique moyenne de 20 à 200 et en particulier de 20 à 50.

De tels polymères sont décrits plus particulièrement dans le brevet US 4 185 087.

Un polymère entrant dans cette classe est le polymère vendu par la Société UNION CARBIDE sous la dénomination "UCAR SILICONE ALE 563".

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit vendu sous la dénomination "EMULSION CATIONIQUE DC 929" par la Société DOW CORNING qui comprend, outre l'amodiméthicone, un agent de surface cationique comprenant un mélange de produits répondant à la formule : 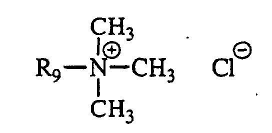 dans lequel R₉ désigne des radicaux alcényle et/ou alcoyle ayant de 14 à 22 atomes de carbone, dérivés des acides gras du suif, en association avec un agent de surface non ionique de formule:

C₉H₁₉-C₆H₄-(OC₂H₄)₁₀⁻OH

connu sous la dénomination "NONOXYNOL 10".

Un autre produit commercial utilisable selon l'invention est le produit vendu sous la dénomination "DOW CORNING Q2 7224" par la Société DOW CORNING comportant en association le triméthylsilylamodiméthicone de formule (IV), un agent de surface non ionique de formule: C₈H₁₇-C₆H₄- (OCH₂CH₂)ₙ-OH où n = 40 dénommé encore "OCTOXYNOL-40", un autre agent de surface non ionique de formule: C₁₂H₂₅-(OCH₂-CH₂)ₙ-OH où n = 6 encore dénommé "ISOLAURETH-6", et du glycol .

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone-acrylate ou -méthacrylate de dialkylaminoalkyle quaternisés ou non, tels que les produits vendus sous la dénomination "GAFQUAT" par la Société ISP, comme par exemple "GAFQUAT 734, GAFQUAT 755 ou GAFQUAT HS100" ou bien les produits dénommés "COPOLYMER 937" ou "COPOLYMER 845". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société UNION CARBIDE CORPORATION. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylamidopropyl triméthyl ammonium ou de diméthyl-diallylammonium.
   Les produits commercialisés répondant à cette définition sont plus particulièrement les produits vendus sous la dénomination "CELQUAT L 200" et "CELQUAT H 100" par la Société NATIONAL STARCH.
(4) Les polysaccharides et notamment gommes de guar cationiques décrits plus particulièrement dans les brevets US 3 589 578 et 4 031 307 et plus particulièrement les produits commercialisés sous les dénominations "JAGUAR C 13 S", "JAGUAR C 15", "JAGUAR C 17" vendus, par la Société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE F", "CARTARETINE F4" ou "CARTARETINE F8" par la société SANDOZ.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "HERCOSETT 57" par la société HERCULES Inc. ou bien sous la dénomination "PD 170" ou "DELSETTE 101" par la société HERCULES dans le cas du copolymère d'acide adipique/ époxypropyl/diéthylène-triamine.
(9) Les cyclopolymères de méthyl diallyl amine ou de diméthyl diallyl ammonium tels que les polymères comportant comme constituant de la chaîne des motifs répondant aux formules (VI) ou (VI'): 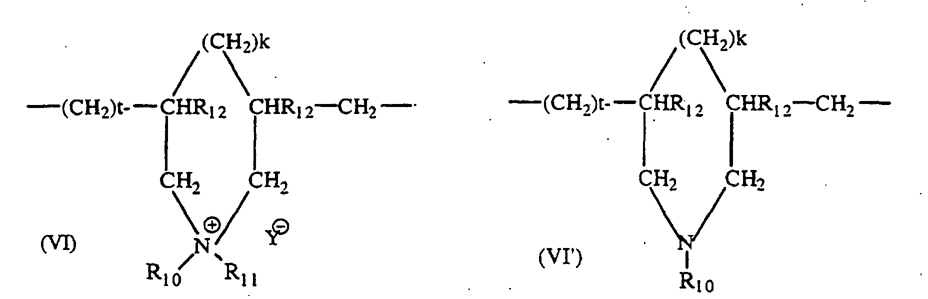 formules dans lesquelles k et t sont égaux à 0 ou 1, la somme k + t étant égale à 1; R₁₂ désigne un atome d'hydrogène ou un radical méthyle; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle; Y^{⊖} est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "MERQUAT 100" par la société MERCK, les copolymères de chlorure de diméthyldiallylammonium et d'acrylamide vendu sous la dénomination "MERQUAT 550".
(10) Le polymère de diammonium quaternaire contenant des motifs récurrents répondant à la formule : 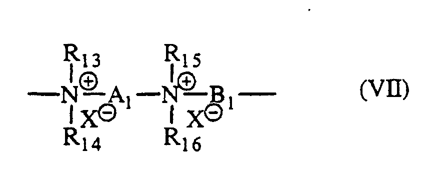 formule (VII) dans laquelle:
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X^{⊖} désigne un anion dérivé d'un acide minéral ou organique;
   A₁, R₁₃ et R₁₅ peuvent former avec les deux atomes d'azote auxquels ils sont rattachés un cycle pipérazinique; en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₙ dans lequel D désigne:
      a) un reste de glycol de formule: -O-Z-O-, où Z désigne un radical hydrocarboné linéaire ou ramifié ou un groupement répondant à l'une des formules suivantes:

         -(CH₂-CH₂-O)ₓ-CH₂-CH₂

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen;
      b) un reste de diamine bis-secondaire tel qu'un dérivé de pipérazine;
      c) un reste de diamine bis-primaire de formule: -NH-Y-NH, où Y désigne un radical hydrocarboné linéaire ou ramifié, ou bien le radical bivalent

         -CH₂-CH₂-S-S-CH₂-CH₂- ;
      d) un groupement uréylène de formule: -NH-CO-NH-;

      De préférence, X^{⊖} est un anion tel que le chlorure ou le bromure.
      Ces polymères ont une masse moléculaire généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) Les polymères de polyammonium quaternaire constitués de motifs de formule (VIII): 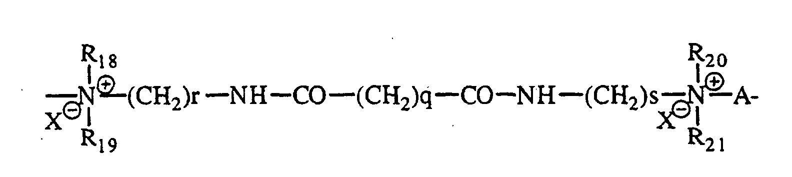 formule dans laquelle:
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer. parmi ceux-ci, les produits "MIRAPOL A 15", "MIRAPOL 10 AD1", "MIRAPOL AZ1" et "MIRAPOL 175" vendus par la société MIRANOL.
(12) Les homopolymères ou copolymères dérivés des acides acrylique ou méthacrylique et comportant des motifs: 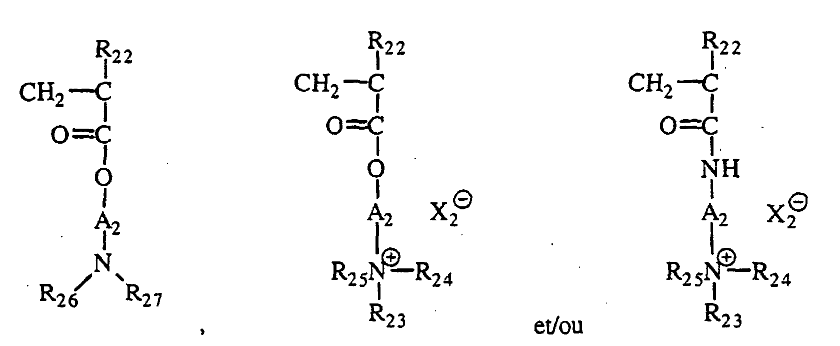 dans lesquels les groupements R₂₂ désignent indépendamment H ou CH₃,
   les groupements A₂ désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignent indépendamment un groupe alcoyle de 1 à 18 atomes de carbone ou un radical benzyle,
   les groupements R₂₆ et R₂₇ représentent un atome d'hydrogène ou un groupement alcoyle de 1 à 6 atomes de carbone,
   X₂^{⊖} désigne un anion, par exemple méthosulfate ou halogénure, tel que chlorure ou bromure.

   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations "LUVIQUAT FC 905", "LUVIQUAT FC 550" et "LUVIQUAT FC 370" par la société BASF.
(14) Les polyamines comme le "POLYQUART H" vendu par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyl triméthylammonium tels que les polymères obtenus par homopolymérisation du diméthylaminoéthylméthacrylate quaternisé par le chloruré de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléhnique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92» par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre dans les produits non rincés les copolymères vinylpyrrolidone-acrylate ou -méthacrylate de dialkylaminoalkyle, quaternisés ou non, tels que les produits vendus sous la dénomination "GAFQUAT" par la société ISP comme par exemple "GUAFQUAT 734, GAFQUAT 755 ou GAFQUAT HS 100" ou encore les produits dénommés "COPOLYMER 937" ou "COPOLYMER 845" vendus aussi par la société ISP et les polymères quaternaires de vinylpyrrolidone et de vinylimidazole tels que les produits commercialisés sous les dénominations "LUVIQUAT FC 905", "LUVIQUAT FC 550" et "LUVIQUAT FC 370" par la société BASF.

Selon l'invention, on peut également utiliser des polymères cationiques sous forme de latex ou de pseudolatex, c'est-à-dire sous forme d'une dispersion de particules de polymères insolubles.

Les polymères cationiques sont utilisés dans les compositions de l'invention dans des proportions comprises entre 0,01 et 20% en poids et de préférence entre 0,1 et 8% en poids par rapport au poids total de la composition.

Les compositions selon l'invention contiennent un milieu aqueux cosmétiquement acceptable. Elles présentent un pH pouvant aller de 3,5 à 11, de préférence compris entre 5,5 et 11 et encore plus préférentiellement entre 5,5 et 8,5.

Le milieu cosmétiquement acceptable des compositions selon l'invention est plus particulièrement constitué d'eau et éventuellement de solvants organiques cosmétiquement acceptables.

Les solvants organiques peuvent représenter de 0,5 à 90% du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple les mono-alcools inférieurs linéaires ou ramifiés ayant de 1 à 8 atomes de carbone, les polyéthylène glycols ayant de 6 à 80 unités d'oxyde d'éthylène et les polyols.

Comme solvants organiques amphiphiles, on peut citer les dérivés de polypropylène glycol (PPG) comme les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle, le malate de dioctyle.

Afin que les compositions cosmétiques de l'invention soient plus agréables à utiliser (plus douces à l'application, plus nourrissantes, plus émollientes), il est possible d'ajouter une phase grasse dans le milieu de ces compositions.

La phase grasse peut représenter jusqu'à 50% du poids total de la composition.

Cette phase grasse peut comporter une huile ou une cire ou leurs mélanges, et peut comprendre également des acides gras, des alcools gras et des esters d'acides gras. Les huiles peuvent être choisies parmi les huiles animales, végétales, minérales ou de synthèse et notamment parmi l'huile de vaseline, l'huile de paraffine, les isoparaffines, les poly-α-oléfines, les huiles fluorées et perfluorées. De même, les cires peuvent être choisies parmi les cires animales, fossiles, végétales, minérales ou de synthèse connues en soi.

Les compositions de l'invention peuvent contenir des adjuvants habituels dans le domaine cosmétique comme d'autres gélifiants et/ou épaississants classiques ; des émulsionnants; des tensio-actifs; des agents hydratants ; des émollients ; des actifs hydrophiles ou lipophiles comme des céramides; des agents anti-radicaux libres ; des séquestrants ; des antioxydants ; des conservateurs ; des agents alcalinisants ou acidifiants ; des parfums ; des charges ; des matières colorantes, des silicones volatiles ou non, modifiées ou non, des réducteurs. Les quantités de ces différents adjuvants sont celles classiquement utilisées dans les domaines considérés.

Bien entendu, le spécialiste veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous. toutes les formes appropriées pour une application topique, notamment sous forme de lotion épaissie, sous forme de gels aqueux ou hydroalcooliques, sous forme de dispersions vésiculaires, sous forme d'émulsions simples ou complexes ((H/E, E/H, H/E/H ou E/H/E) et être de consistance liquide, semi-liquide ou solide telles que des crèmes, des laits, des gels, des gels-crèmes, des pâtes, des sticks et éventuellement être conditionnées en aérosol et se présenter sous la forme de mousses ou de sprays. Ces compositions sont préparées selon les méthodes usuelles.

Les compositions selon l'invention sont de préférence utilisées comme produits capillaires rincés ou non-rincés, notamment pour le lavage, la coloration, le soin, le conditionnement, le défrisage, le maintien de la coiffure ou la mise en forme permanente ou non des cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray ou une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions de l'invention peuvent être aussi utilisées comme produits de soin ou d'hygiène tels que des crèmes de protection, de traitement ou de soin pour le visage, pour les mains ou pour le corps, des laits corporels de protection ou de soin, des lotions, gels ou mousses pour le soin ou le nettoyage de la peau.

Les compositions peuvent également consister en des préparations solides constituant des savons ou des pains de nettoyage.

Les compositions de l'invention peuvent être également utilisées comme produits de soin bucco-dentaire tels que des pâtes dentifrices, des bains de bouche.

Les compositions peuvent être des produits pour le maquillage tels que des crèmes pour le visage, des fonds de teint, des mascaras, des eye-liners, des rouges à lèvres, des vernis à ongles.

Un autre objet de l'invention est un procédé de traitement non-thérapeutique cosmétique de la peau, du cuir cheveu, des cheveux, des cils, des sourcils, des ongles ou des muqueuses, caractérisé par le fait qu'on applique sur le support kératinique une composition telle que définie ci-dessus, selon la technique d'utilisation habituelle de cette composition, par exemple application de crèmes, de gels, de sérums, de lotions, de laits sur la peau, le cuir chevelu ou les muqueuses.

Selon l'invention, on traite plus particulièrement les cheveux.

Les exemples suivants illustrent l'invention sans présenter un caractère limitatif.

### EXEMPLE 1 : Soin capillaire gélifié non-rincé

- Terpolymère acide méthacrylique/méthyl acrylate/ diméthyl métaisopropényl benzyl isocyanate d'alcool béhénylique éthoxylé (40 OE) en dispersion aqueuse à 25% 0,5 g MA
- Copolymère vinylpyrrolidone/chlorure de méthyl vinyl imidazolium (70/30) en solution aqueuse à 40% vendu par la société BASF sous la dénomination commerciale "LUVIQUAT FC 370" 0,5 g MA
- 2-amino 2-méthyl propanol-1 (AMP) pH ajusté à 7.5 qs
- Parfum, conservateur, colorant q.s
- Eau déminéralisée qsp 100 g

On obtient un gel fluide, non pâteux, non gras et s'étalant très bien sur les cheveux. Ce gel donne aux cheveux un toucher doux et une bonne aptitude au démêlage et possède un bon pouvoir fixant.

Si on remplace le terpolymère ci-dessus par la même quantité de polyuréthane "ACRYSOL 44" de ROHM et HAAS, on obtient un produit liquide et trouble.

Si on remplace le terpolymère par le copolymère acide acrylique/acrylate d'alkyle en C₁₀/C₃₀ réticulé "PEMULEN TR1" vendu par GOODRICH, on obtient un gel légèrement pâteux, ayant un pouvoir fixant très médiocre et des propriétés cosmétiques de douceur et toucher beaucoup moins bonnes.

### EXEMPLE 2 : Shampooing

- Lauryl éther sulfate de sodium à 2,2 moles d'oxyde d'éthylène 15 g MA
- Cocoylbétaïne 2,5 g MA
- Terpolymère acide méthacrylique/méthyl acrylate/ diméthyl métaisopropényl benzyl isocyanate d'alcool béhénylique éthoxylé (40 OE) en dispersion aqueuse à 25% 1 g MA
- Chlorure d'hydroxypropylguar triéthyl ammonium vendu sous la dénomination "JAGUAR C13S" par la société MEYHALL 0,1g
- Conservateurs, parfum q.s
- Eau qsp, 100 g
   pH ajusté à 6,5 (NaOH)

Ce shampooing se présente sous l'aspect d'un liquide épaissi. Il possède de bonnes propriétés moussantes et laisse les cheveux lisses et faciles à démêler après le shampooing.

### EXEMPLE COMPARATIF 3 : Shampooing

- Lauryl éther sulfate de sodium à 2,2 moles d'oxyde d'éthylène 15 g MA
- Cocoylbétaïne 2,5 g MA
- Polyuréthane à terminaison alkyle polyéthoxylé en solution à 35% dans un mélange propylène glycol/eau (60/40) ("ACRYSOL 44" vendu par la société ROHM & HAAS) 1 g MA
- Chlorure d'hydroxypropylguar triéthyl ammonium vendu sous la dénomination "JAGUAR C13S" par la société MEYHALL 0,1g
- Conservateurs, parfum q.s
- Eau qsp 100 g
   pH ajusté à 6,5 (NaOH)

Ce shampooing se présente sous l'aspect d'un produit trouble très fluide et possède des propriétés- cosmétiques très inférieures à celles du shampooing selon l'invention : les cheveux séchés sont moins lisses et plus difficiles à démêler.

### EXEMPLE 4 : Gel de coiffage non-rincé

- Copolymère vinylpyrrolidone/diméthylamino éthylméthacrylate quatemisé par le sulfate de diéthyle à 50% dans l'éthanol, vendu sous la dénomination "GUAFQUAT 734" par la société ISP 1 g MA
- Terpolymère acide méthacrylique/méthyl acrylate/diméthyl métaisopropényl benzyl isocyanate d'alcool béhénylique éthoxylé (40 OE) en dispersion aqueuse à 25% 1 g MA
- Ethanol absolu 8.7 g
- Parfum, conservateur, colorant q.s
- 2-amino 2-méthyl propanol-1 (AMP) pH ajusté à 7.5 qs
- Eau déminéralisée qsp 100 g

On obtient un gel fluide, non pâteux, non gras, s'étalant bien sur les cheveux. Ce gel donne aux cheveux un toucher doux, une bonne aptitude au démêlage et possède un bon pouvoir fixant.

## Revendications

1. Composition cosmétique destinée au traitement des matières kératiniques et notamment des cheveux, **caractérisée par le fait qu'**elle comprend, dans un support aqueux cosmétiquement acceptable, au moins un polymère cationique et un terpolymère acrylique comprenant :
a) 20 à 70% en poids, et de préférence 25 à 55% en poids, d'un acide carboxylique à insaturation α, β-monoéthylénique ;
b) 20 à 80% en poids, et de préférence 30 à 65% en poids, d'un monomère à insaturation monoéthylénique non tensio-actif différent de a) et
c) 0,5 à 60% en poids, et de préférence 10 à 50% en poids, d'un monomère uréthane non-ionique qui est le produit de réaction d'un tensio-actif non-ionique monohydrique avec un monoisocyanate à insaturation monoéthylénique.

2. Composition selon la revendication 1, **caractérisée par le fait que** l'acide carboxylique à insaturation α, β-monoéthylénique a) est choisi parmi l'acide acrylique, l'acide méthacrylique, l'acide itaconique et l'acide maléique.

3. Composition selon la revendication 2, **caractérisée par le fait que** l'acide carboxylique à insaturation α, β-monoéthylénique a) est l'acide méthacrylique.

4. Composition selon l'une quelconque des revendications 1 à 3, **caractérisée par le fait que** le monomère à insaturation monoéthylénique non tensio-actif b) est choisi parmi les acrylates et méthacrylates d'alkyle en C₁-C₄. le styrène, le vinyltoluène, l'acétate de vinyle, l'acrylonitrile et le chlorure de vinylidène.

5. Composition selon la revendication 4, **caractérisée par le fait que** le monomère à insaturation monoéthylénique non tensio-actif est l'acrylate de méthyle ou d'éthyle.

6. Composition selon l'une quelconque des revendications 1 à 5, **caractérisée par le fait que** le tensio-actif non-ionique monohydrique utilisé pour obtenir le monomère uréthane non-ionique c) a pour formule : 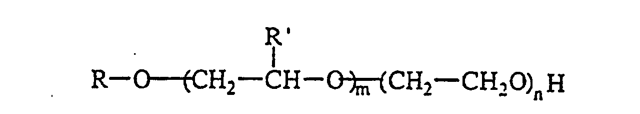 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

7. Composition selon la revendication 6, **caractérisée par le fait que** R est choisi parmi les radicaux dodécyle, alkyle en C₁₈-C₂₆ et alkyl (C₈-C₁₃) phényle, m = 0 et n est un nombre moyen allant d'environ 5 à 150.

8. Composition selon l'une quelconque des revendications 1 à 7, **caractérisée par le fait que** le monoisocyanate à insaturation monoéthylénique utilisé pour former le monomère uréthane non-ionique c) est l'α,α-diméthyl m-isopropényl benzyl isocyanate.

9. Composition selon l'une quelconque des revendications 1 à 8, **caractérisée par le fait que** le terpolymère acrylique est obtenu en dispersion aqueuse à partir d'acide méthacrylique comme composant a), d'acrylate de méthyle comme composant b) et d'un macromonomère uréthane non-ionique de structure suivante : 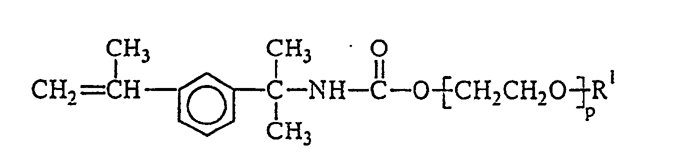 dans laquelle p va de 6 à 150 et R¹ est un radical alkyle en C₁₈-C₂₆ de préférence en C₂₀-C₂₄ linéaire, d'origine végétale, tel que le radical docosyle.

10. Composition selon l'une quelconque des revendications 1 à 9, **caractérisée par le fait que** le terpolymère acrylique est présent dans des concentrations allant de 0,01 à 20% en poids, et de préférence de 0,1 à 10 % en poids, par rapport au poids total de la composition.

11. Composition selon l'une quelconque des revendications 1 à 10, **caractérisée par le fait que** les polymères cationiques sont choisis parmi les protéines ou hydrolysats de protéines quaternisés, les polymères cationiques siliconés, les copolymères vinylpyrrolidone-acrylate ou -méthacrylate de dialkylaminoalkyle quaternisés ou non, les dérivés d'éthers de cellulose comportant des groupes ammonium quaternaire, les dérivés de cellulose cationiques, les polysaccharides cationiques, les polymères constitués de motifs pipérazinyle et de radicaux divalents alkylène ou hydroxyalkylène, les polyaminoamides solubles dans l'eau préparés par polycondensation d'un composé acide avec une polyamine, les dérivés de polyaminoamides résultant de la condensation de polyalcoylène polyamines avec des acides polycarboxyliques suivie d'une alcoylation par des agents bifonctionnels, les polymères obtenus par réaction d'une polyalkylène polyamine comportant deux groupements amine primaire et au moins un groupement amine secondaire avec un acide dicarboxylique, les cyclopolymères de méthyl diallyl amine ou de diméthyl diallyl ammonium, les polymères de diammonium quaternaire, les polymères de polyammonium quaternaire, les homopolymères ou copolymères dérivés des acides acrylique ou méthacrylique, comportant des motifs esters ou amides substitués par un groupement contenant une fonction amine ou ammonium quaternaire, les polymères quaternaires de vinylpyrrolidone et de vinylimidazole, les polyamines, les polymères réticulés de chlorure de méthacryloyloxyéthyl triméthylammonium et leurs mélanges.

12. Composition selon la revendication 11, **caractérisée par le fait qu'**elle est de type non-rincé et comprend de préférence un ou plusieurs polymères cationiques choisis parmi les copolymères vinylpyrrolidone-acrylate ou -méthacrylate de dialkylaminoalkyle quaternisés ou non et les polymères quaternaires de vinylpyrrolidone et de vinylimidazole.

13. Composition selon l'une quelconque des revendications 1 à 12, **caractérisée par le fait que** le ou les polymères cationiques sont présents dans des concentrations allant de 0,01 à 20% en poids, et de préférence de 0,1 à 8% en poids, par rapport au poids total de la composition.

14. Composition selon l'une quelconque des revendications 1 à 13, **caractérisée par le fait qu'**elle présente un pH allant de 3,5 à 11, de préférence de 5,5 à 11 et encore plus préférentiellement de 5,5 à 8,5.

15. Composition selon l'une quelconque des revendications 1 à 14, **caractérisée par le fait que** le milieu aqueux cosmétiquement acceptable est constitué d'eau ou d'eau et au moins un solvant organique choisi dans le groupe constitué par les solvants organiques hydrophiles, lipophiles, amphiphiles ou leurs mélanges.

16. Composition selon l'une quelconque des revendications 1 à 15, **caractérisée par le fait qu'**elle comprend en outre au moins un adjuvant cosmétique usuel choisi parmi les corps gras, les gélifiants et/ou épaississants classiques, les tensio-actifs, les agents hydratants, les émollients, les actifs hydrophiles ou lipophiles comme les céramides, les agents anti-radicaux libres, les séquestrants, les antioxydants, les conservateurs, les agents alcalinisants ou acidifiants, les parfums, les charges, les matières colorantes, les silicones et les réducteurs.

17. Composition selon l'une quelconque des revendications 1 à 16, **caractérisée par le fait qu'**elle se présente sous forme d'émulsion, de lotion, de gel, de dispersion vésiculaire, de pâte, de bâtonnet solide ou est conditionnée en aérosol et se présente sous forme de mousse ou de spray.

18. Composition selon l'une quelconque des revendications 1 à 17, **caractérisée par le fait qu'**elle est utilisée comme produit capillaire rincé ou non-rincé pour le lavage, la teinture, le soin, le conditionnement, le défrisage, le maintien de la coiffure ou la mise en forme permanente ou non des cheveux.

19. Procédé de traitement non-thérapeutique cosmétique pour le traitement des matières kératiniques et plus particulièrement des cheveux, **caractérisé par le fait qu'**on applique sur ces derniers une quantité efficace d'une composition telle que définie selon l'une quelconque des revendications 1 à 18.

## Patentansprüche

1. Kosmetische Zusammensetzung, die zur Behandlung von Keratinsubstanzen und insbesondere Haaren vorgesehen ist, **dadurch gekennzeichnet, daß**
sie in einem kosmetisch akzeptablen wäßrigen Träger mindestens ein kationisches Polymer und ein Acrylterpolymer enthält, welches enthält:
a) 20 bis 70 Gew.-% und vorzugsweise 25 bis 55 Gew.-% einer Carbonsäure mit α,β-monoethylenischer Doppelbindung;
b) 20 bis 80 Gew.-% und vorzugsweise 30 bis 65 Gew.-% eines Monomers mit monethylenischer Doppelbindung, das kein grenzflächenaktiver Stoff und von a) verschieden ist, und
c) 0,5 bis 60 Gew.-% und vorzugsweise 10 bis 50 Gew.-% eines nichtionischen Urethanmonomers, das das Produkt der Umsetzung eines nichtionischen grenzflächenaktiven Stoffes mit einer Hydroxygruppe und eines Monoisocyanats mit monoethylenischer Doppelbindung ist.

2. Zusammensetzung nach Anspruch 1, **dadurch gekennzeichnet, daß** die Carbonsäure mit α,β-monoethylenischer Doppelbindung a) unter Acrylsäure, Methacrylsäure, Itaconsäure und Maleinsäure ausgewählt ist.

3. Zusammensetzung nach Anspruch 2, **dadurch gekennzeichnet, daß** die Carbonsäure mit α,β-monoethylenischer Doppelbindung a) die Methacrylsäure ist.

4. Zusammensetzung nach einem der Ansprüche 1 bis 3, **dadurch gekennzeichnet, daß** das Monomer mit monoethylenischer Doppelbindung b), das kein grenzflächenaktiver Stoff ist, unter den C₁₋₄-Alkyl-acrylaten und C₁₋₄-Alkyl-methacrylaten, Styrol, Vinyltoluol, Vinylacetat, Acrylnitril und Vinylidenchlorid ausgewählt ist.

5. Zusammensetzung nach Anspruch 4, **dadurch gekennzeichnet, daß** das Monomer mit monoethylenischer Doppelbindung, das kein grenzflächenaktiver Stoff ist, Methylacrylat oder Ethylacrylat ist.

6. Zusammensetzung nach einem der Ansprüche 1 bis 5, **dadurch gekennzeichnet, daß** der zur Herstellung des nichtionischen Urethanmonomers c) verwendete nichtionische grenzflächenaktive Stoff mit einer Hydroxygruppe die folgende Formel aufweist: 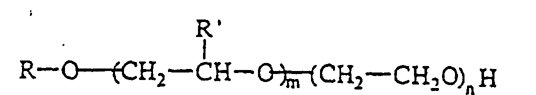 worin R eine C₆₋₃₀-Alkylgruppe oder C₈₋₃₀-Aralkylgruppe, R' eine C₁₋₄-Alkylgruppe, n eine Zahl von 5 bis 150 und m eine Zahl von 0 bis 50 bedeutet, mit der Maßgabe, daß n mindestens so groß wie m ist und n + m = 5 - 150.

7. Zusammensetzung nach Anspruch 6, **dadurch gekennzeichnet, daß** R unter Dodecyl, C₁₈₋₂₆-Alkyl und C₈₋₁₃-Alkylphenyl ausgewählt ist und m = 0 und n eine Zahl von etwa 5 bis 150 bedeutet.

8. Zusammensetzung nach einem der Ansprüche 1 bis 7, **dadurch gekennzeichnet, daß** das Monoisocyanat mit monoethylenischer Doppelbindung, das zur Bildung des nichtionischen Urethanmonomers c) verwendet wird, das α,α-Dimethyl-m-isopropenylbenzylisocyanat ist.

9. Zusammensetzung nach einem der Ansprüche 1 bis 8, **dadurch gekennzeichnet, daß** das Acrylterpolymer in wäßriger Dispersion ausgehend von Methacrylsäure als Komponente a), Methylacrylat als Komponente b) und einem nichtionischen Urethanmakromer der folgenden Struktur hergestellt wird: 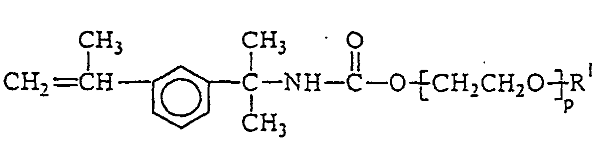 wobei p im Bereich von 6 bis 150 liegt und R¹ eine Alkylgruppe mit 18 bis 26 Kohlenstoffatomen und vorzugsweise eine geradkettige Alkylgruppe mit 20 bis 24 Kohlenstoffatomen pflanzlicher Herkunft ist, wie Docosyl.

10. Zusammensetzung nach einem der Ansprüche 1 bis 9, **dadurch gekennzeichnet, daß** das Acrylterpolymer in Konzentrationen von 0,01 bis 20 Gew.-% und vorzugsweise 0,1 bis 10 Gew.-%, bezogen auf das Gesamtgewicht der Zusammensetzung, vorliegt.

11. Zusammensetzung nach einem der Ansprüche 1 bis 10, **dadurch gekennzeichnet, daß** die kationischen Polymere ausgewählt sind unter: quaternisierten Proteinen oder Proteinhydrolysaten, kationischen Siliconpolymeren, Copolymeren von Vinylpyrrolidon und Dialkylaminoalkylacrylat oder -methacrylat, die gegebenenfalls quaternisiert sind, Celluloseetherderivaten, die quartäre Ammoniumgruppen enthalten, kationischen Cellulosederivaten, kationischen Polysacchariden, Polymeren, die aus Piperazinyleinheiten und zweiwertigen Alkylen- oder Hydroxyalkylengruppen bestehen, in Wasser löslichen Polyaminoamiden, die durch Polykondensation einer Säure mit einem Polyamin hergestellt sind, Polyaminoamidderivaten, die bei der Kondensation von Polyalkylenpolyamiden mit Polycarbonsäuren und anschließender Alkylierung mit bifunktionellen Mitteln entstehen, Polymeren, die durch Umsetzung eines Polyalkylenpolyamins mit zwei primären Aminogruppen und mindestens einer sekundären Aminogruppe mit einer Carbonsäure hergestellt sind, Methyldiallylamin-Cyclopolymeren oder Dimethyldiallylammonium-Cyclopolymeren, quartären Diammoniumpolymeren, quartären Polyammoniumpolymeren, Homopolymeren oder Copolymeren, die von Acrylsäure oder Methacrylsäure abgeleitet sind und Ester- oder Amideinheiten enthalten, die mit einer Gruppe substituiert sind, die eine Aminogruppe oder quartäre Ammoniumgruppe enthält, quartären Polymeren von Vinylpyrrolidon und Vinylimidazol, Polyaminen, vernetzten Polymeren von Methacryloyloxyethyltrimethylammoniumchlorid und deren Gemischen.

12. Zusammensetzung nach Anspruch 11, **dadurch gekennzeichnet, daß** sie im Haar verbleibt und vorzugsweise ein oder mehrere kationische Polymere enthält, die unter den Copolymeren von Vinylpyrrolidon und Dialkylaminoalkylacrylat oder -methacrylat, die gegebenenfalls quaternisiert sind, und den quartären Polymeren von Vinylpyrrolidon und Vinylimidazol ausgewählt sind.

13. Zusammensetzung nach einem der Ansprüche 1 bis 12, **dadurch gekennzeichnet, daß** das kationische Polymer oder die kationischen Polymere in Konzentrationen von 0,01 bis 20 Gew.-% und vorzugsweise 0,1 bis 8 Gew.-%, bezogen auf das Gesamtgewicht der Zusammensetzung, vorliegen.

14. Zusammensetzung nach einem der Ansprüche 1 bis 13, **dadurch gekennzeichnet, daß** sie einen pH-Wert von 3,5 bis 11 und vorzugsweise 5,5 bis 11 und noch bevorzugter 5,5 bis 8,5 aufweisen.

15. Zusammensetzung nach einem der Ansprüche 1 bis 14, **dadurch gekennzeichnet, daß** das kosmetisch akzeptable Medium aus Wasser oder Wasser und mindestens einem organischen Lösungsmittel besteht, das unter den hydrophilen, lipophilen, amphiphilen organischen Lösungsmitteln oder deren Gemischen ausgewählt ist.

16. Zusammensetzung nach einem der Ansprüche 1 bis 15, **dadurch gekennzeichnet, daß** sie mindestens einen herkömmlichen kosmetischen Zusatzstoff enthält, der unter den Fettsubstanzen, herkömmlichen Gelbildnern und/oder Verdickungsmitteln, grenzflächenaktiven Stoffen, Hydratisierungsmitteln, Emollentien, hydrophilen oder lipophilen Wirkstoffen, wie Ceramiden, Mitteln gegen freie Radikale, Maskierungsmitteln, Antioxidantien, Konservierungsmitteln, Alkalisierungsmitteln oder Ansäuerungsmitteln, Parfums, Füllstoffen, Farbmitteln, Siliconen und Reduktionsmitteln ausgewählt ist.

17. Zusammensetzung nach einem der Ansprüche 1 bis 16, **dadurch gekennzeichnet, daß** sie als Emulsion, Lotion, Gel, Vesikeldispersion, Paste oder fester Stift vorliegt oder als Aerosol konfektioniert ist und in Form von Schaum oder Spray vorliegt.

18. Zusammensetzung nach einem der Ansprüche 1 bis 17, **dadurch gekennzeichnet, daß** sie als Produkt für das Haar, das ausgespült wird oder im Haar verbleibt, zum Waschen, Färben, Pflegen, Konditionieren, Entkräuseln der Haare und für den Halt der Frisur oder die Formgebung der Haare, die dauerhaft oder nicht dauerhaft sein können, verwendet wird.

19. Verfahren zur nicht therapeutischen kosmetischen Behandlung von Keratinfasern und insbesondere der Haare, **dadurch gekennzeichnet, daß** auf diese eine Zusammensetzung nach einem der Ansprüche 1 bis 18 in einer wirksamen Menge aufgebracht wird.

## Claims

1. Cosmetic composition intended for the treatment of keratinous material and in particular the hair, **characterized in that** it comprises, in a cosmetically acceptable aqueous support, at least one cationic polymer and an acrylic terpolymer comprising:
a) 20 to 70% by weight, and preferably 25 to 55% by weight, of a carboxylic acid containing α,β-monoethylenic unsaturation;
b) 20 to 80% by weight, and preferably 30 to 65% by weight, of a non-surfactant monomer containing monoethylenic unsaturation, which is different from a), and
c) 0.5 to 60% by weight, and preferably 10 to 50% by weight, of a nonionic urethane monomer which is the product of reaction of a monohydric nonionic surfactant with a monoisocyanate containing monoethylenic unsaturation.

2. Composition according to Claim 1, **characterized in that** the carboxylic acid containing α,β-monoethylenic unsaturation a) is chosen from acrylic acid, methacrylic acid, itaconic acid and maleic acid.

3. Composition according to Claim 2, **characterized in that** the carboxylic acid containing α,β-monoethylenic unsaturation a) is methacrylic acid.

4. Composition according to any one of Claims 1 to 3, **characterized in that** the non-surfactant monomer containing monoethylenic unsaturation b) is chosen from C₁-C₄ alkyl acrylates and methacrylates, styrene, vinyltoluene, vinyl acetate, acrylonitrile and vinylidene chloride.

5. Composition according to Claim 4, **characterized in that** the non-surfactant monomer containing monoethylenic unsaturation is methyl or ethyl acrylate.

6. Composition according to any one of Claims 1 to 5, **characterized in that** the monohydric nonionic surfactant used to obtain the nonionic urethane monomer c) has the formula: 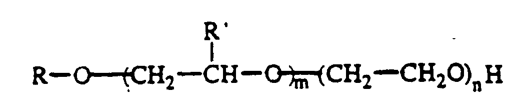 in which R is a C₆-C₃₀ alkyl or C₈-C₃₀ aralkyl group, R' is a C₁-C₄ alkyl group, n is an average number ranging from 5 to 150 and m is an average number ranging from 0 to 50, with the condition that n is at least as large as m and that n+m = 5-150.

7. Composition according to Claim 6, **characterized in that** R is chosen from dodecyl, C₁₈-C₂₆ alkyl and (C₈-C₁₃) alkylphenyl radicals, m = 0 and n is an average number ranging from about 5 to 150.

8. Composition according to any one of Claims 1 to 7, **characterized in that** the monoisocyanate containing monoethylenic unsaturation which is used to form the nonionic urethane monomer c) is α,α-dimethyl-m-isopropenyl benzyl isocyanate.

9. Composition according to any one of Claims 1 to 8, **characterized in that** the acrylic terpolymer is obtained as an aqueous dispersion from methacrylic acid as component a), methyl acrylate as component b) and a nonionic urethane macromonomer of the following structure: 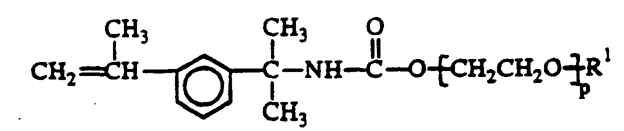 in which p ranges from 6 to 150 and R¹ is a C₁₈-C₂₆, preferably C₂₀-C₂₄, linear alkyl radical of plant origin, such as the docosyl radical.

10. Composition according to any one of Claims 1 to 9, **characterized in that** the acrylic terpolymer is present in concentrations ranging from 0.01 to 20% by weight, and preferably from 0.1 to 10% by weight, relative to the total weight of the composition.

11. Composition according to any one of Claims 1 to 10, **characterized in that** the cationic polymers are chosen from quaternized proteins or protein hydrolysates, silicone-based cationic polymers, quaternized or non-quaternized vinylpyrrolidone/ dialkylaminoalkyl acrylate or methacrylate copolymers, cellulose ether derivatives containing quaternary ammonium groups, cationic cellulose derivatives, cationic polysaccharides, polymers consisting of piperazinyl units and of divalent alkylene or hydroxyalkylene radicals, water-soluble polyaminoamides prepared by polycondensation of an acidic compound with a polyamine, polyaminoamide derivatives resulting from the condensation of polyalkylenepolyamines with polycarboxylic acids followed by an alkylation with difunctional agents, polymers obtained by reaction of a polyalkylenepolyamine containing two primary amine groups and at least one secondary amine group with a dicarboxylic acid, methyldiallylamine or dimethyldiallylammonium cyclopolymers, quaternary diammonium polymers, polyquaternary ammonium polymers, homopolymers or copolymers derived from acrylic or methacrylic acid, containing ester or amide units substituted with a group containing an amine or quaternary ammonium function, quaternary vinylpyrrolidone and vinylimidazole polymers, polyamines, methacryloyloxyethyltrimethylammonium chloride crosslinked polymers and mixtures thereof.

12. Composition according to Claim 11, **characterized in that** it is of leave-in type and preferably comprises one or more cationic polymers chosen from quaternized or non-quaternized vinylpyrrolidone/ dialkylaminoalkyl acrylate or methacrylate copolymers and quaternary vinylpyrrolidone and vinylimidazole polymers.

13. Composition according to any one of Claims 1 to 12, **characterized in that** the cationic polymer(s) is (are) present in concentrations ranging from 0.01 to 20% by weight, and preferably from 0.1 to 8% by weight, relative to the total weight of the composition.

14. Composition according to any one of Claims 1 to 13, **characterized in that** it has a pH ranging from 3.5 to 11, preferably from 5.5 to 11 and even more preferably from 5.5 to 8.5.

15. Composition according to any one of Claims 1 to 14, **characterized in that** the cosmetically acceptable aqueous medium consists of water or of water and at least one organic solvent chosen from the group consisting of hydrophilic, lipophilic and amphiphilic organic solvents or mixtures thereof.

16. Composition according to any one of Claims 1 to 15, **characterized in that** it also comprises at least one common cosmetic adjuvant chosen from fatty substances, standard gelling agents and/or thickeners, surfactants, moisturizers, emollients, hydrophilic or lipophilic active agents such as ceramides, anti-free-radical agents, sequestering agents, antioxidants, preserving agents, acidifying or basifying agents, fragrances, fillers, dyestuffs, silicones and reducing agents.

17. Composition according to any one of Claims 1 to 16, **characterized in that** it is in the form of an emulsion, a lotion, a gel, a vesicle dispersion, a paste or a solid stick or is packaged as an aerosol and is in the form of a mousse or a spray.

18. Composition according to any one of Claims 1 to 17, **characterized in that** it is used as a rinse-out or leave-in hair product to wash, dye, care for, condition or straighten the hair, to maintain the hairstyle or to permanently or temporarily reshape the hair.

19. Cosmetic, non-therapeutic treatment process for treating keratinous material, and more particularly the hair, **characterized in that** an effective amount of a composition as defined according to any one of Claims 1 to 18 is applied to the hair.
